(19) Europäisches Patentamt
European Patent Office
Office européen des brevets

(11) EP 1 418 163 A1

(12) EUROPÄISCHE PATENTANMELDUNG

(43) Veröffentlichungstag:
**12.05.2004 Patentblatt 2004/20**

(51) Int Cl.[7]: **C07C 17/20**, C07C 19/14

(21) Anmeldenummer: **03024920.5**

(22) Anmeldetag: **29.10.2003**

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**AL LT LV MK**

(30) Priorität: **11.11.2002 DE 10252272**

(71) Anmelder: **Bayer Chemicals AG
51368 Leverkusen (DE)**

(72) Erfinder:
- **Marhold, Albrecht, Dr.
51373 Leverkusen (DE)**
- **Pleschke, Axel, Dr.
51069 Köln (DE)**

(74) Vertreter: **Zobel, Manfred, Dr. et al
Bayer Chemicals AG,
Law & Patents,
Patents and Licensing
51368 Leverkusen (DE)**

(54) **Verfahren zur Herstellung von Polyhalogenalkanen**

(57) Die vorliegende Erfindung betrifft ein Verfahren zur Herstellung von Polyhalogenalkanen und deren Anwendung zur Herstellung von Zwischenprodukten für Agrochemikalien und Arzneimitteln.

EP 1 418 163 A1

## Beschreibung

Die vorliegende Erfindung betrifft ein Verfahren zur Herstellung von Polyhalogenalkanen und deren Anwendung zur Herstellung von Zwischenprodukten für Agrochemikalien und Arzneimittel.

Polyhalogenalkane wie insbesondere Perfluoralkylchloride, -bromide und -iodide sind wertvolle Ausgangsprodukte insbesondere zur Herstellung von polyhalogenalkylierten Aromaten. Polyhalogenalkylierte Aromaten gewinnen zunehmend an Bedeutung, da sie als lipophile Bausteine in Agro- oder Pharmawirkstoffen für eine gute Membrangängigkeit sorgen.

Es ist zum Beispiel aus US-Patent 3,770,838 bekannt, Polyfluoralkylchloride und -bromide durch Umsetzung von Polyfluoralkenen mit Kaliumfluorid und Chlor- oder Bromcyan herzustellen. Die hohe Toxizität der Halogencyane ist jedoch in der technischen Anwendung unerwünscht. Weiterhin ist aus US-Patent 5,057,634 bekannt, aus $C_3$-Bausteinen durch gemischte Gasphasen-Chlorierung und -Fluorierung mit Chlor und Fluorwasserstoff unter anderem 2-Chlor-heptafluorpropan herzustellen. Die geringe Chemoselektivität und die drastischen Bedingungen sind unvorteilhaft. Petrov et al. (J. Fluorine Chem., 2000, 102, 199-204) beschreiben ein Verfahren zur Herstellung von Perfluoralkylhalogeniden durch Umsetzung von Perfluoralkenen mit Perfluoralkylhalogeniden in Gegenwart von Aluminiumsalzen. Der hohe Bedarf an diesen Aluminiumsalzen macht eine technische Anwendung des Verfahrens uninteressant.

Es bestand daher weiterhin das Bedürfnis, ein Verfahren bereitzustellen, das die Herstellung von Polyfluoralkylchloriden, -bromiden und -iodiden in guten Ausbeuten und moderaten Reaktionsbedingungen in einfacher Weise ermöglicht.

Es wurde nun ein Verfahren zur Herstellung von Verbindungen der Formel (I) gefunden,

$$R^1R^2CHal^1\text{-}CFR^3R^4 \qquad (I)$$

in der

| | |
|---|---|
| $Hal^1$ | für Chlor, Brom oder Iod |
| $R^1$, $R^2$, $R^3$ und $R^4$ | jeweils unabhängig voneinander für Wasserstoff, Fluor, Chlor, Brom oder $C_1$-$C_{12}$-Halogenalkyl stehen oder jeweils zwei der Reste $R^1$, $R^2$, $R^3$ und $R^4$ zusammen mindestens einen cyclischen Rest mit insgesamt 4 bis 8 Ringatomen bilden |

das dadurch gekennzeichnet ist, dass
Verbindungen der Formel (II),

$$R^1R^2CHal^1\text{-}CHal^2R^3R^4 \qquad (II)$$

in der

| | |
|---|---|
| $Hal^1$ und $Hal^2$ | jeweils unabhängig voneinander die für $Hal^1$ vorstehend genannte Bedeutung besitzen und |
| $R^1$, $R^2$, $R^3$ und $R^4$ | jeweils unabhängig die vorstehend genannte Bedeutung besitzen |

in Gegenwart von ionischem Fluorid umgesetzt werden.

Im Rahmen der Erfindung können alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Weise kombiniert werden.

**Halogenalkyl** steht jeweils unabhängig für einen geradkettigen, cyclischen, verzweigten oder unverzweigten Alkyl-Rest, der einfach, mehrfach oder vollständig durch Fluor- und/oder Chloratome substituiert ist. Unter dem Begriff Halogenalkyl sind auch mehrfach durch Fluoratome und gegebenenfalls einfach oder mehrfach durch Chlor substituierte Alkylreste, im Folgenden Polyfluoralkyl-Reste genannt, und vollständig durch Fluoratome substituierte Alkylreste, im Folgenden Perfluoralkyl-Reste genannt, umfasst.

Beispielsweise steht $C_1$-$C_4$-Halogenalkyl für Trifluormethyl, 2,2,2-Trifluorethyl, Pentafluorethyl, Nonafluorbutyl und Difluorchlormethyl, $C_1$-$C_{12}$-Halogenalkyl darüberhinaus beispielsweise für Perfluorcyclohexyl, Perfluor-n-pentyl, Perfluor-n-hexyl und Perfluor-n-dodecyl.

Bevorzugt stehen

Hal[1] und Hal[2] jeweils unabhängig für Brom oder Chlor, besonders bevorzugt identisch für Brom.

$R^1$, $R^2$, $R^3$ und $R^4$ stehen bevorzugt jeweils unabhängig für Wasserstoff, Fluor, Chlor, $C_1$-$C_4$-Polyfluoralkyl oder $C_1$-$C_4$-Perfluoralkyl, besonders bevorzugt jeweils unabhängig für Wasserstoff, Fluor, Chlor oder $C_1$-$C_4$-Perfluoralkyl.

Besonders bevorzugt stehen jeweils drei oder vier Reste aus $R^1$, $R^2$, $R^3$ und $R^4$ für Fluor oder $C_1$-$C_4$-Perfluoralkyl und kein oder ein Rest für Wasserstoff oder Chlor.

Bevorzugte Verbindungen der Formel (I) sind 1,2-Dibromtetrafluorethan, 1,2-Dibrom-1-chlor-trifluorethan, 2,3-Dibrom-octafluorbutan, 2,3-Dibrom-2,3-dichlorhexafluorbutan, 2,3-Dibrom-2,3-dichlorhexafluorbutan, 2,3-Dibrom-1,1,1,3,4,4,4-heptafluorbutan, 2,3-Dibrom-2-chlor-1,1,1,4,4,4-hexafluorbutan, 1,2-Dibromhexafluorpropan und 1,2-Dichlorhexafluorpropan, wobei 1,2-Dibromhexafluorpropan, 2-Dibrom-1-chlor-trifluorethan besonders bevorzugt sind. Ganz besonders bevorzugt ist 1,2-Dibromhexafluorpropan.

Besonders bevorzugt eignet sich das erfindungsgemäße Verfahren zur Herstellung von 1-Brompentafluorethan aus 1,2-Dibromtetrafluorethan, 1-Brom-2-chlor-tetrafluorethan und 1-Chlor-2-brom-tetrafluorethan aus 1,2-Dibrom-1-chlor-trifluorethan, 2-Brom-nonafluorbutan aus 2,3-Dibrom-octafluorbutan, 2-Brom-2,3-dichlor-heptafluorbutan aus 2,3-Dibrom-2,3-dichlorhexafluorbutan, 2-Brom-1,1,1,3,3,4,4,4-octafluorbutan und 2-Brom-1,1,1,2,3,4,4,4-octafluorbutan aus 2,3-Dibrom-1,1,1,3,4,4,4-heptafluorbutan, 2-Brom-2-chlor-1,1,1,3,4,4,4-heptafluorbutan und 2-Brom-3-chlor-1,1,1,3,4,4,4-heptafluorbutan aus 2,3-Dibrom-2-chlor-1,1,1,4,4,4-hexafluorbutan und 2-Brom-heptafluorpropan aus 1,2-Dibromhexafluorpropan oder 2-Chlor-heptafluorpropan aus 1-Brom-2-chlorhexafluorpropan oder 1,2-Dichlorhexafluorpropan.

Die Umsetzung von Verbindungen der Formel (II) erfolgt in Gegenwart von ionischem Fluorid.

Ionische Fluoride sind beispielsweise quartäre Ammonium- oder Phosphoniumfluoride sowie Alkalimetallfluoride oder Mischungen der genannten Verbindungen.

Beispiele für Ammonium- oder Phosphoniumfluoride sind solche der Formel (III),

$$(Kation^+)(F^-) \qquad (III)$$

in der

$(Kation^+)$ für Kationen der Formel (IV) steht

$$[Pnyc(C_1\text{-}C_{12}\text{-Alkyl})_q(C_6\text{-}C_{15}\text{-Arylalkyl})_r(C_5\text{-}C_{14}\text{-Aryl})_S(\{(C_2\text{-}C_6\text{-Alkyl})\text{-O}]_v\text{-}(C_1\text{-}C_6\text{-Alkyl})\}_t)]^+ \qquad (IV)$$

wobei

Pnyc für Stickstoff oder Phosphor steht und

in der (q+r+s+t) = 4 ist.

Bevorzugt ist jedoch der Einsatz von Alkalimetallfluoriden oder Mischungen von Alkalimetallfluoriden, wobei Natrium-, Kalium- und Cäsiumfluorid besonders bevorzugt und Kaliumfluorid ganz besonders bevorzugt ist. Vorzugsweise setzt man Kaliumfluorid ein, das einen möglichst geringen Wassergehalt und eine möglichst große Oberfläche aufweist.

Das molare Verhältnis von ionischem Fluorid zu eingesetzter Verbindung der Formel (II) kann beispielsweise 0,7 bis 5 betragen, vorzugsweise 0,9 bis 2 und besonders bevorzugt 1,1 bis 1,7. Bei Einsatz von Verbindungen der Formel (II), die keine weiteren Chloratome besitzen, ist die Menge an ionischem Fluorid nach oben prinzipiell nicht begrenzt, größere Mengen sind aber unwirtschaftlich.

Es wurde gefunden, dass üblicherweise Bromatome vor Chloratomen substituiert werden und bei gleichem Atomtyp die Substitutionsgeschwindigkeit in der Reihe primäres, sekundäres, tertiäres Kohlenstoffatom drastisch abnimmt. Man beobachtet beispielsweise bei der Reaktion von 1,2-Dibromhexafluorpropan mit einer Selektivität von größer 20:1 die Substitution am primären Kohlenstoffatom.

Vorzugsweise wird das Verfahren in einem organischen Lösungsmittel durchgeführt. Als organische Lösungsmittel sind beispielsweise geeignet: Ketone, wie Aceton, 2-Butanon oder Methyl-isobutyl-keton; Nitrile, wie Acetonitril, Propionitril, Benzonitril, Benzylnitril oder Butyronitril; Amide, wie N,N-Dimethylformamid, N,N-Dimethylacetamid, N-Methyl-formanilid, N-Methyl-pyrrolidon, N-Methylcaprolactam oder Hexamethylphosphorsäuretriamid; Sulfoxide, wie Dimethylsulfoxid, Sulfone wie Tetramethylensulfon, Polyether wie 1,4-Dioxan, Ethylenglykoldimethylether, Ethylenglykoldiethylether, Diethylenglykoldimethylether, Diethylenglykoldiethylether oder Gemische solcher organischen Lösungsmittel.

Vorzugsweise beträgt der Wassergehalt des Lösungsmittels im erfindungsgemäßen Verfahren maximal 1 Gew.-%, bevorzugt maximal 0,2 Gew.-%. Vorzugsweise wird ein solcher Wassergehalt durch Andestillieren oder Trocknung in an sich bekannter Weise erreicht. Bei Einsatz von Alkalimetallfluoriden wird besonders bevorzugt das Lösungsmittel gleichzeitig in Gegenwart des eingesetzten Alkalimetallfluorids getrocknet bzw. andestilliert.

Die Reaktionstemperatur kann beispielsweise 60°C bis zum Siedepunkt des eingesetzten Lösungsmittel bei Reaktionsdruck betragen, maximal jedoch 300°C, bevorzugt 110°C bis zum Siedepunkt des eingesetzten Lösungsmittel bei Reaktionsdruck maximal jedoch 200°C betragen.

Der Reaktionsdruck kann beispielsweise 0,8 bis 100 bar betragen, bevorzugt sind 3 bis 25 bar.

Die Reaktionsdauer kann beispielsweise 10 min bis 72 Stunden, bevorzugt 2 bis 12 Stunden betragen.

Gegebenenfalls kann die Reaktivität der ionischen Fluoride durch Zusätze modifiziert werden. Geeignete Zusätze sind beispielsweise Phasentransferkatalysatoren und/oder Halex-Katalysatoren.

Als Phasentransferkatalysatoren sind beispielsweise Kronenether, wie 18-Krone-6, 12-Krone-4, Dibenzo-18-Krone-6 oder Dibenzo-12-Krone-4, Kryptanden wie Kryptand[2.2.2] oder Podanden wie Polyglykolether oder solche der Formel (V) geeignet,

$$(\text{Kation}^+)(\text{Anion}^-) \qquad \text{(V)}$$

in der

(Kation$^+$) vorstehend genannte Bedeutung und Vorzugsbereiche besitzt und

(Anion$^-$) für das Anion einer organischen oder anorganischen Säure steht.

Halex-Katalysatoren sind beispielsweise Tetrakis(dialkylamino)phosphoniumverbindungen (WO 98/05610) oder Verbindungen der Formel (VI),

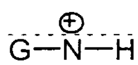 An$^{\ominus}$ (VI),

in der

G für einen Rest der Formeln (VIIa) oder (VIIb) steht

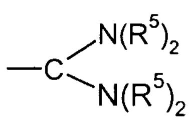

(VIIa)

$-P\{N(R^5)_2\}_3$

(VIIb)

und

H unabhängig von G für einen Rest der Formeln (VIIa), (VIIb), (VIIc) oder (VIId)

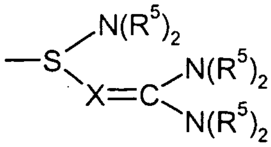

(VIIc)

$$-S[N(R^5)_2]_2 \qquad \text{(VIId)}$$

stehen,
wobei die Reste

$R^5$ jeweils unabhängig voneinander für $C_1$-$C_{12}$-Alkyl, $C_2$-$C_{10}$-Alkenyl oder $C_6$-$C_{12}$-Aryl stehen

oder wobei
$N(R^5)_2$ als Ganzes für einen 3- bis 5-gliedrigen, gesättigten oder ungesättigten Ring stehen kann,
oder wobei die Reste der Formel (VIIa) und/oder die Gruppe

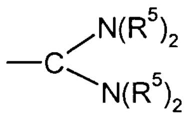

(VIIa)

als Ganzes für einen gesättigten oder ungesättigten 4- bis 8-gliedrigen Rings stehen kann, und

X für Stickstoff oder Phosphor steht und

$An^{\ominus}$ für ein Äquivalent eines Anions wie beispielsweise und bevorzugt Chlorid, Bromid, $(CH_3)_3SiF_2^{\ominus}$, $HF_2^{\ominus}$, $H_2F_2^{\ominus}$, Tetrafluoroborat, Hexafluorophosphat, Carbonat oder Sulfat steht.

Die Verbindungen der Formel (VI) sind beispielsweise erhältlich durch Umsetzung von Verbindungen der Formel (VIII),

$$[G\text{-}An']^{\oplus}An^{\ominus} \qquad \text{(VIII)}$$

in der

G und $An^{\ominus}$ die bei Formel (VI) angegebene Bedeutung besitzen und

An' für Chlor oder Brom und steht,

mit Verbindungen der Formel (IX),

$$HN{=}G' \qquad \text{(IX)}$$

in der

G' hinsichtlich der Anordnung der Atome die bei Formel (VI) für G angegebene Bedeutung hat, jedoch 2-bindig ist, wobei die Umsetzung in Gegenwart einer Base erfolgt.

Die Verbindungen der Formel (VI) sind beschrieben in DE 101 29 057.

Die Aufarbeitung des in Schritt a) erhaltenen Reaktionsgemisches kann in an sich bekannter Weise erfolgen, üblicherweise beispielsweise durch fraktionierte Destillation direkt aus dem Reaktionsgemisch, gegebenenfalls unter vermindertem Druck.

Die als Ausgangsprodukte verwendeten Verbindungen der Formel (II) sind literaturbekannt oder analog zur Literatur synthetisierbar. In einer bevorzugten Ausführungsform werden die Verbindungen der Formel (II) dadurch hergestellt, dass
Verbindungen der Formel (X)

$$R^1\text{-}CR^2\text{=}CR^3\text{-}R^4 \qquad (X)$$

mit Halogenverbindungen der Formel (XI),

$$Hal^1\text{-}Hal^2 \qquad (XI)$$

in denen
$Hal^1$ und $Hal^2$ die unter der Formel (II) vorstehend genannte Bedeutung besitzen, umgesetzt werden.

Besonders bevorzugte Verbindungen der Formel (XI) sind Chlor und Brom, wobei Brom noch weiter bevorzugt ist.

Die Reaktion kann mit oder ohne Lösungsmittel in an sich bekannter Weise durchgeführt werden. Bevorzugt ist die Durchführung ohne Lösungsmittel. Beispielsweise kann man die Halogenverbindung vorlegen und gegebenenfalls unter Kühlung die Verbindung der Formel (X) zugeben.

Die Reaktionstemperatur kann beispielsweise -40 bis 100°C, bevorzugt 0 bis 40°C und ganz besonders bevorzugt 15 bis 40°C betragen.

Das molare Verhältnis von Verbindungen der Formel (XI) zu Verbindungen der Formel (III) kann beispielsweise 0,5 bis 3, vorzugsweise 0,8 bis 1,5 und besonders bevorzugt 0,9 bis 1,2 betragen. Größere und kleinere Mengen sind möglich, aber unwirtschaftlich.

Die erfindungsgemäß erhältlichen Verbindungen der Formel (I) eignen sich insbesondere in einem Verfahren zur Herstellung von Agrochemikalien und Arzneimitteln oder Zwischenprodukten davon, bevorzugt zur Herstellung von polyfluoralkylierten aromatischen Verbindungen.

Ein wesentlicher Vorteil der erfindungsgemäß erhältlichen Verbindungen der Formel (I) ist, dass sie in einfacher Weise in hohen Ausbeuten aus gut verfügbaren Edukten hergestellt werden können.

## Beispiele

### Beispiel 1

**Herstellung von 1,2-Dibrom-hexafluorpropan**

Es wurden bei Raumtemperatur 2357 g Brom (760 ml, 14,75 mol) vorgelegt und unter stetigem Rühren Hexafluorpropen bis zur Entfärbung eingeleitet (19 Stunden, 2400 g, 16,00 mol). Das Reaktionsgemisch wurde mit Stickstoff ausgeblasen. Auf diese Weise wurden 4710 g 1,2-Dibrom-hexafluorpropan (95 % d.Th.) erhalten.

### Beispiel 2

**Herstellung von 1,2-Dibrom-1-chlor-trifluorethan**

Es wurden bei Raumtemperatur 155,1 g Brom (50 ml, 0,97 mol) vorgelegt und unter stetigem Rühren Trifluorchlorethylen bis zur Entfärbung eingeleitet (4 Stunden, 113 g, 0,97 mol). Das Reaktionsgemisch wurde mit Stickstoff ausgeblasen. Auf diese Weise wurden 260 g 1,2-Dibrom-1-chlor-trifluorethan (96 % d.Th.) erhalten.

### Beispiel 3

**Herstellung von 2-Brom-heptafluorpropan (a)**

In einem Autoklaven wurden Tetramethylensulfon (2450 ml) und 352 g Kaliumfluorid (6,05 mol) vorgelegt und der Ansatz durch Abdestillieren von 250 ml Lösungsmittel getrocknet. Anschließend wurden 1250 g 1,2-Dibrom-hexafluorpropan aus Beispiel 1 zugegeben, der Ansatz unter 3 bar Stickstoff gesetzt und auf 125°C aufgeheizt, wobei sich ein Druck von 13,5 bar einstellte. Es wurde bei gleicher Temperatur noch zwei Stunden erhitzt und dann die

Temperatur in drei Stunden auf 175°C gesteigert. Es wurde auf 0°C abgekühlt, entspannt und das Produkt aus dem Reaktionsgemisch in eine Kühlfalle destilliert. Auf diese Weise wurden 870 g 2-Brom-heptafluorpropan mit einer Reinheit von 95,8 % erhalten (83 % d.Th.).

## Beispiel 4

### Herstellung von 2-Brom-heptafluorpropan (b)

In einem 40 1 Autoklaven wurden Tetramethylensulfon (14300 ml) und 5541 g geglühtes Kaliumfluorid (95,38 mol) vorgelegt und der Ansatz durch Abdestillieren von 1500 ml Lösungsmittel getrocknet. Anschließend wurden bei 120°C 19700 g 1,2-Dibrom-hexafluorpropan (63,58mol) aus Beispiel 1 zugegeben, der Ansatz unter Stickstoff gesetzt und auf 125°C aufgeheizt, wobei sich ein Druck von 10,7 bar einstellte. Es wurde bei gleicher Temperatur noch fünf Stunden erhitzt. Es wurde auf 30°C abgekühlt, entspannt und das Produkt aus dem Reaktionsgemisch in eine Kühlfalle destilliert. Auf diese Weise wurden 15168 g 2-Brom-heptafluorpropan mit einer Reinheit von 99,2 % erhalten (95 % d. Th.).

## Patentansprüche

1. Verfahren zur Herstellung von Verbindungen der Formel (I),

$$R^1R^2CHal^1\text{-}CFR^3R^4 \quad \text{(I)}$$

in der

| | |
|---|---|
| $Hal^1$ | für Chlor, Brom oder Iod |
| $R^1$, $R^2$, $R^3$ und $R^4$ | jeweils unabhängig voneinander für Wasserstoff, Fluor, Chlor, Brom oder $C_1$-$C_{12}$-Halogenalkyl stehen oder jeweils zwei der Reste $R^1$, $R^2$, $R^3$ und $R^4$ zusammen mindestens einen cyclischen Rest mit insgesamt 4 bis 8 Ringatomen bilden |

**dadurch gekennzeichnet, dass**
Verbindungen der Formel (II),

$$R^1R^2CHal^1\text{-}CHal^2R^3R^4 \quad \text{(II)}$$

in der

| | |
|---|---|
| $Hal^1$ und $Hal^2$ | jeweils unabhängig voneinander die für $Hal^1$ vorstehend genannte Bedeutung besitzen und |
| $R^1$, $R^2$, $R^3$ und $R^4$ | jeweils unabhängig die vorstehend genannte Bedeutung besitzen |

in Gegenwart von ionischem Fluorid umgesetzt werden.

2. Verfahren nach Anspruch 1, **dadurch gekennzeichnet, dass** $Hal^1$ und $Hal^2$ jeweils unabhängig für Brom oder Chlor stehen.

3. Verfahren nach mindestens einem der Ansprüche 1 bis 2, **dadurch gekennzeichnet, dass** $R^1$ und $R^4$ jeweils unabhängig für Fluor, Chlor, $C_1$-$C_4$-Polyfluoralkyl oder $C_1$-$C_4$-Perfluoralkyl stehen.

4. Verfahren nach mindestens einem der Ansprüche 1 bis 3, **dadurch gekennzeichnet, dass** $R^2$ und $R^3$ jeweils unabhängig für Wasserstoff, Fluor, Chlor, $C_1$-$C_4$-Polyfluoralkyl oder $C_1$-$C_4$-Perfluoralkyl stehen.

5. Verfahren nach mindestens einem der Ansprüche 1 bis 4, **dadurch gekennzeichnet, dass** als Verbindungen der Formel (II) 1,2-Dibromtetrafluorethan, 1,2-Dibrom-1-chlor-trifluorethan, 2,3-Dibrom-octafluorbutan, 2,3-Dibrom-2,3-dichlorhexafluorbutan, 2,3-Dibrom-2,3-dichlorhexafluorbutan, 2,3-Dibrom-1,1,1,3,4,4,4-heptafluorbutan, 2,3-Dibrom-2-chlor-1,1,1,4,4,4-hexafluorbutan, 1,2-Dibromhexafluorpropan oder 1,2-Dichlorhexafluorpropan eingesetzt werden.

**6.** Verfahren nach mindestens einem der Ansprüche 1 bis 5, **dadurch gekennzeichnet, dass** 1-Brompentafluorethan aus 1,2-Dibromtetrafluorethan, 1-Brom-2-chlor-tetrafluorethan und 1-Chlor-2-brom-tetrafluorethan aus 1,2-Dibrom-1-chlor-trifluorethan, 2-Brom-nonafluorbutan aus 2,3-Dibrom-octafluorbutan, 2-Brom-2,3-dichlor-heptafluorbutan aus 2,3-Dibrom-2,3-dichlorhexafluorbutan, 2-Brom-1,1,1,3,3,4,4,4-octafluorbutan und 2-Brom-1,1,1,2,3,4,4,4-octafluorbutan aus 2,3-Dibrom-1,1,1,3,4,4,4-heptafluorbutan, 2-Brom-2-chlor-1,1,1,3,4,4,4-heptafluorbutan und 2-Brom-3-chlor-1,1,1,3,4,4,4-heptafluorbutan aus 2,3-Dibrom-2-chlor-1,1,1,4,4,4-hexafluorbutan und 2-Brom-heptafluorpropan aus 1,2-Dibromhexafluorpropan oder 2-Chlor-heptafluorpropan aus 1-Brom-2-chlorhexafluorpropan oder 1,2-Dichlorhexafluorpropan hergestellt werden.

**7.** Verfahren nach mindestens einem der Ansprüche 1 bis 6, **dadurch gekennzeichnet, dass** 2-Brom-heptafluorpropan aus 1,2-Dibromhexafluorpropan hergestellt wird.

**8.** Verfahren nach mindestens einem der Ansprüche 1 bis 7, **dadurch gekennzeichnet, dass** als ionische Fluoride quartäre Ammonium- oder Phosphoniumfluoride oder Alkalimetallfluoride oder Mischungen davon eingesetzt werden.

**9.** Verfahren nach mindestens einem der Ansprüche 1 bis 8, **dadurch gekennzeichnet, dass** Natrium-, Kalium- und/ oder Cäsiumfluorid eingesetzt werden.

**10.** Verfahren nach mindestens einem der Ansprüche 1 bis 9, **dadurch gekennzeichnet, dass** es in einem organischen Lösungsmittel durchgeführt wird.

**11.** Verfahren nach Anspruch 10, **dadurch gekennzeichnet, dass** der Wassergehalt des Lösungsmittels maximal 1 Gew.-% beträgt.

**12.** Verfahren nach mindestens einem der Ansprüche 1 bis 11, **dadurch gekennzeichnet, dass** die Reaktionstemperatur beispielsweise 70°C bis zum Siedepunkt des eingesetzten Lösungsmittel bei Reaktionsdruck beträgt, wobei der Reaktionsdruck 1 bis 100 bar beträgt.

**13.** Verfahren nach mindestens einem der Ansprüche 1 bis 12, **dadurch gekennzeichnet, dass** die Reaktivität der ionischen Fluoride durch Zusätze modifiziert wird.

**14.** Verfahren nach mindestens einem der Ansprüche 1 bis 13, **dadurch gekennzeichnet, dass** die als Ausgangsprodukte verwendeten Verbindungen der Formel (II) dadurch hergestellt werden, dass
Verbindungen der Formel (X)

$$R^1\text{-}CR^2\text{=}CR^3\text{-}R^4 \qquad (X)$$

mit Halogenverbindungen der Formel (XI),

$$Hal^1\text{-}Hal^2 \qquad (XI)$$

in denen

$Hal^1$ und $Hal^2$ die unter der Formel (II) vorstehend genannte Bedeutung besitzen, umgesetzt werden.

**15.** Verfahren nach Anspruch 14, **dadurch gekennzeichnet, dass** als Verbindungen der Formel (XI) Brom eingesetzt wird.

**16.** Verfahren nach mindestens einem der Ansprüche 14 bis 15, **dadurch gekennzeichnet, dass** die Reaktion ohne Lösungsmittel durchgeführt wird.

17. Verwendung von Verbindungen der Formel (I), die gemäß einem Verfahren nach mindestens einem der Ansprüche 1 bis 16 hergestellt wurden in einem Verfahren zur Herstellung von Agrochemikalien und Arzneimitteln oder Zwischenprodukten davon.

18. Verwendung nach Anspruch 17, **dadurch gekennzeichnet, dass** polyfluoralkylierte aromatische Verbindungen hergestellt werden.

Europäisches Patentamt

# EUROPÄISCHER RECHERCHENBERICHT

Nummer der Anmeldung
EP 03 02 4920

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | GB 965 643 A (DOW CORNING CORPORATION) 6. August 1964 (1964-08-06) * Seite 1, Zeile 39 - Seite 3, Zeile 52; Ansprüche 1-5; Beispiele 1-3,5-8 * | 1-16 | C07C17/20 C07C19/14 |
| X | WO 91 12225 A (E.I. DU PONT DE NEMOURS AND COMPANY) 22. August 1991 (1991-08-22) * Seite 4, Zeile 1 - Seite 4, Zeile 35 * * Seite 6, Zeile 17 - Seite 8, Zeile 30 * * Seite 10, Zeile 32 - Seite 11, Zeile 13 * | 1-14 | |
| X | EP 0 936 212 A (NIHON NOHYAKU CO., LTD) 18. August 1999 (1999-08-18) * Seite 2, Zeile 5 - Seite 3, Zeile 27 * * Seite 5, Zeile 48 - Seite 10, Zeile 35 * | 17,18 | |
| X | EP 1 006 102 A (NIHON NOHYAKU CO., LTD) 7. Juni 2000 (2000-06-07) * Seite 1, Zeile 5 - Seite 1, Zeile 19 * * Seite 3, Zeile 35 - Seite 6, Zeile 46 * * Seite 10, Zeile 5 - Seite 21, Zeile 25 * | 17,18 | RECHERCHIERTE SACHGEBIETE (Int.Cl.7) |
| P,A | DE 101 29 057 A (BAYER AG) 19. Dezember 2002 (2002-12-19) * Beispiel 2 * | 13 | C07C |

Der vorliegende Recherchenbericht wurde für alle Patentansprüche erstellt

| Recherchenort | Abschlußdatum der Recherche | Prüfer |
|---|---|---|
| MÜNCHEN | 17. Februar 2004 | Cooper, S |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 03 02 4920

*In diesem Anhang sind die Mitglieder der Patentfamilien der im obengenannten europäischen Recherchenbericht angeführten Patentdokumente angegeben.*
Die Angaben über die Familienmitglieder entsprechen dem Stand der Datei des Europäischen Patentamts am
*Diese Angaben dienen nur zur Unterrichtung und erfolgen ohne Gewähr.*

17-02-2004

| Im Recherchenbericht angeführtes Patentdokument | | Datum der Veröffentlichung | Mitglied(er) der Patentfamilie | | | Datum der Veröffentlichung |
|---|---|---|---|---|---|---|
| GB 965643 | A | 06-08-1964 | DE | 1443696 | A1 | 05-12-1968 |
| | | | FR | 1357392 | A | 03-04-1964 |
| WO 9112225 | A | 22-08-1991 | US | 4990702 | A | 05-02-1991 |
| | | | AU | 7180391 | A | 03-09-1991 |
| | | | CN | 1054059 | A | 28-08-1991 |
| | | | DE | 69108903 | D1 | 18-05-1995 |
| | | | DE | 69108903 | T2 | 16-11-1995 |
| | | | EP | 0515402 | A1 | 02-12-1992 |
| | | | ES | 2071296 | T3 | 16-06-1995 |
| | | | JP | 5504138 | T | 01-07-1993 |
| | | | WO | 9112225 | A1 | 22-08-1991 |
| | | | ZA | 9010403 | A | 27-05-1992 |
| EP 0936212 | A | 18-08-1999 | CN | 1228413 | A | 15-09-1999 |
| | | | EP | 0936212 | A1 | 18-08-1999 |
| | | | JP | 11302233 | A | 02-11-1999 |
| | | | US | 2003055287 | A1 | 20-03-2003 |
| | | | US | 2001041814 | A1 | 15-11-2001 |
| EP 1006102 | A | 07-06-2000 | AU | 731777 | B2 | 05-04-2001 |
| | | | AU | 5937499 | A | 01-06-2000 |
| | | | EP | 1380568 | A2 | 14-01-2004 |
| | | | EP | 1006102 | A2 | 07-06-2000 |
| | | | HU | 9904445 | A2 | 28-02-2001 |
| | | | JP | 2001122836 | A | 08-05-2001 |
| | | | KR | 2000035767 | A | 26-06-2000 |
| | | | TW | 513393 | B | 11-12-2002 |
| | | | US | 2002198399 | A1 | 26-12-2002 |
| | | | US | 2003204104 | A1 | 30-10-2003 |
| | | | CN | 1257861 | A | 28-06-2000 |
| DE 10129057 | A | 19-12-2002 | DE | 10129057 | A1 | 19-12-2002 |
| | | | EP | 1266904 | A1 | 18-12-2002 |
| | | | JP | 2003064033 | A | 05-03-2003 |
| | | | US | 2003036667 | A1 | 20-02-2003 |

EPO FORM P0461

Für nähere Einzelheiten zu diesem Anhang : siehe Amtsblatt des Europäischen Patentamts, Nr.12/82